(19) Europäisches Patentamt
European Patent Office
Office européen des brevets

(11) Veröffentlichungsnummer: **0 184 110**
**A2**

(12) # EUROPÄISCHE PATENTANMELDUNG

(21) Anmeldenummer: 85114962.5

(22) Anmeldetag: 26.11.85

(51) Int. Cl.⁴: C 07 C 148/00

(30) Priorität: 06.12.84 DE 3444494

(43) Veröffentlichungstag der Anmeldung: 11.06.86 Patentblatt 86/24

(84) Benannte Vertragsstaaten: AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Arndt, Michael, Dr.
Cäcilienstrasse 5
D-5600 Wuppertal 1(DE)

(72) Erfinder: Sehnem, Hans Peter, D.I.
Heinrichstrasse 7
D-5830 Schwelm(DE)

(54) **Verfahren zur Herstellung von Alkylthioacetamidinen.**

(57) Es wird ein neues Verfahren zur Herstellung von Alkylthioacetamidinen der Formel (I)

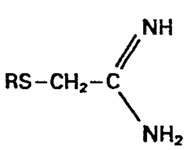 x $(HX)_n$ (I)

bereitgestellt,
worin
R = Alkyl
X = Halogen
und
n = 0 oder 1.

Das neue Verfahren ist dadurch gekennzeichnet, daß man

(a) Chloracetonitril der Formel (II)

$ClCH_2CN$ (II)

mit Ammoniumhalogeniden der Formel (III)

$NH_4X$ (III)

in welcher
X die oben angegebene Bedeutung hat,
in Gegenwart von Katalysatoren und in Gegenwart von Verdünnungsmitteln bei Temperaturen zwischen −20°C und +30°C zu den Chloracetamidinen der Formel (IV)

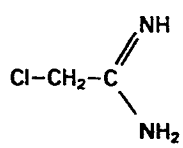 x $(HX)_n$ (IV)

in welcher
X und n die oben angegebenen Bedeutungen haben,
umsetzt und

(b) diese Verbindungen der Formel (IV) gegebenenfalls ohne Isolierung mit Alkylmercaptanen der Formel (V)

RSH (V)

in welcher
R die oben angegebene Bedeutung hat,
in Gegenwart von Säureakzeptoren und in Gegenwart von Verdünnungsmitteln, bei Temperaturen zwischen −20°C und +30°C und einem Druck zwischen 1 und 10 bar zu den Verbindungen der Formel (I) umsetzt.

Bei den Verbindungen der Formel (I) handelt es sich um Zwischenprodukte für die Synthese von hochwirksamen Insektiziden.



Croydon Printing Company Ltd.

0184110

BAYER AKTIENGESELLSCHAFT
Konzernverwaltung RP
Patentabteilung

5090 Leverkusen, Bayerwerk

Er/Kü-c

Typ IV a

## Verfahren zur Herstellung von Alkylthioacetamidinen

Die Erfindung betrifft ein neues Verfahren zur Herstellung von Alkylthioacetamidinen aus Chloracetonitril und Alkylmercaptanen in Gegenwart einer alkoholischen Alkoholatlösung.

Es ist bereits bekannt, daß man Alkoxyalkyl-Derivate herstellen kann, indem man Alkoholate mit Halogenalkyl-Derivaten umsetzt (vgl. z.B. "Methoden der organischen Chemie" Band VI/3, S. 26, Houben-Weyl-Müller, Thieme-Verlag-Stuttgart). Außerdem ist bekannt, daß man Alkylthioalkyl-Derivate aus den entsprechenden Alkylmercaptanen und Halogenalkyl-Derivaten herstellen kann (vgl. z.B. "Methoden der organischen Chemie", Band IX, S. 103, Houben-Weyl-Müller, Thieme-Verlag-Stuttgart).

Es wurde nun gefunden, daß man Alkylthioacetamidine der Formel (I)

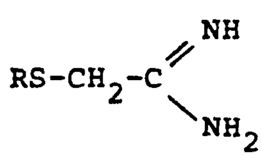 x $(HX)_n$ (I)

in welcher

R für Alkyl steht,

X für Halogen steht und

n für 0 oder 1 steht,

erhält, wenn man

(a) Chloracetonitril der Formel (II)

$ClCH_2CN$ (II)

mit Ammoniumhalogeniden der Formel (III)

$NH_4X$ (III)

in welcher

X die oben angegebene Bedeutung hat,

in Gegenwart von Katalysatoren und in Gegenwart von Verdünnungsmitteln bei Temperaturen zwischen -20°C und +30°C zu den Chloracetamidinen der Formel (IV)

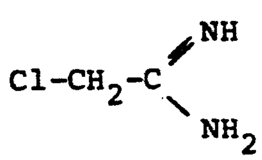 x $(HX)_n$ (IV)

in welcher

X und n die oben angegebenen Bedeutungen haben,

umsetzt und

(b) diese Verbindungen der Formel (IV) gegebenenfalls ohne Isolierung mit Alkylmercaptanen der Formel (V)

RSH (V)

in welcher

R die oben angegebene Bedeutung hat,

in Gegenwart von Säureakzeptoren und in Gegenwart von Verdünnungsmitteln, bei Temperaturen zwischen -20°C und +30°C und einem Druck zwischen 1 und 10 bar zu den Verbindungen der Formel (I) umsetzt.

Überraschenderweise ist es möglich mit Hilfe des erfindungsgemäßen Verfahrens die Umsetzung in kurzen Reaktionszeiten und mit hohen Ausbeuten durchzuführen, obwohl nach dem Stand der Technik die Bildung von Alkoxyacetamidinen im beträchtlichem Ausmaß hätte erwartet werden müssen. Als vorteilhaft erweist sich bei diesem Verfahren, daß eine Belastung der Abluft mit Alkylmercaptanen durch die Verwendung von geschlossenen Reaktoren weitgehend vermieden wird.

Bevorzugt wird die Umsetzung zur Herstellung der Verbindungen der Formel (I) ohne Isolierung der Zwischenstufe (IV) durchgeführt.

Bevorzugt werden mit dem erfindungsgemäßen Verfahren die Verbindungen der Formel (I) hergestellt, in welcher

R für Alkyl mit 1 bis 6 Kohlenstoffatomen steht,

X für Chlor oder Brom steht und

n für 0 oder 1 steht.

Besonders bevorzugt werden mit dem erfindungsgemäßen Verfahren die Verbindungen der Formel (I) hergestellt, in welcher

R für Methyl, Ethyl, n-Propyl, i-Propyl, n-Butyl, i-Butyl, sec.-Butyl und tert.-Butyl steht,

X für Chlor steht und

n für 0 oder 1 steht.

Verwendet man für das erfindungsgemäße Verfahren Chloracetonitril, Ammoniumchlorid und Methylmercaptan als Ausgangsstoffe, so kann die Reaktion durch das folgende Formelschema wiedergegeben werden:

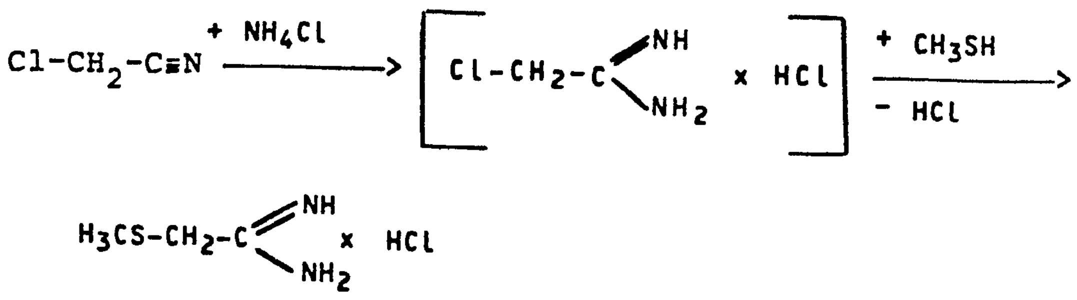


Die Verbindung der Formel (II), die als Ausgangsstoff für die Durchführung des erfindungsgemäßen Verfahrens benötigt wird, ist eine allgemein bekannte Verbindung der organischen Chemie.

Die weiterhin als Ausgangsstoffe für das erfindungsgemäße Verfahren benötigten Verbindungen sind durch die Formeln (III), (IV) und (V) allgemein definiert. In diesen Formeln stehen X und n bzw. R für diejenigen Reste, welche oben bei der Definition der Verbindungen der Formel (I) genannt sind.

Als Beispiele für die Verbindungen der Formel (III) seien genannt:

Ammoniumchlorid, -bromid und -iodid.

Als Beispiele für die Verbindungen der Formel (IV) seien genannt:

Chloracetamidin, Chloracetamidin-Hydrochlorid und Chloracetamidin-Hydrobromid.

Als Beispiele für die Verbindungen der Formel (V) seien genannt:

Methyl-, Ethyl-, n-Propyl-, i-Propyl-, n-Butyl-, i-Butyl-, sec.-Butyl- und tert.-Butylmercaptan.

Die Verbindungen der Formeln (III), (IV) und (V) sind allgemein bekannte Verbindungen der organischen Chemie.

Das erfindungsgemäße Verfahren wird in beiden Reaktionsschritten in Gegenwart von Verdünnungsmitteln durchgeführt. Als solche kommen unter den Reaktionsbedingungen inerte Lösungsmittel in Frage. Hierzu gehören insbesondere Alkohole, wie Methanol, Ethanol und n-Propanol. Bevorzugt wird Methanol als Lösungsmittel eingesetzt.

Das erfindungsgemäße Verfahren wird in Stufe (a) in Gegenwart von Katalysatoren durchgeführt. Als Katalysatoren kommen vorzugsweise in Frage: Alkalialkoholate, wie Natrium- oder Kaliummethylat und Natrium- oder Kaliumethylat. Bevorzugt werden Natriummethylat oder -ethylat als Katalysator eingesetzt.

Das erfindungsgemäße Verfahren wird in Stufe (b) in Gegenwart von Säureakzeptoren durchgeführt. Als Säureakzeptoren kommen vorzugsweise die obengenannten Alkalialkoholate in Betracht. Bevorzugt werden Natriummethylat oder -ethylat als Säureakzeptor eingesetzt. Als Verdünnungsmittel wird beim Einsetzen von Natriummethylat Methanol, und beim Einsatz von Natriumethylat Ethanol eingesetzt.

Das erfindungsgemäße Verfahren wird in beiden Stufen im allgemeinen bei Temperaturen zwischen -20°C und +30°C, vorzugsweise zwischen -10°C und +20°C durchgeführt. Die Reaktionen werden vorzugsweise in einer Inertgasatmosphäre durchgeführt. Als Inertgas kommt vorzugsweise Argon oder Stickstoff in Betracht. Bevorzugt wird in Gegenwart von Stickstoff gearbeitet.

Das erfindungsgemäße Verfahren wird in Stufe (a) bevorzugt unter Normaldruck durchgeführt. Stufe (b) wird im allgemeinen bei einem Druck zwischen 1 und 10 bar, vorzugsweise zwischen 1 und 5 bar durchgeführt.

Bei der Durchführung des erfindungsgemäßen Verfahrens wird in Stufe (a) auf 1 Mol Chloracetonitril der Formel (II) 0,01 bis 0,2 Mol, vorzugsweise 0,05 bis 0,15 Mol Katalysator und 1 bis 2,00 Mol, vorzugsweise 1 bis 1,5 Mol Ammoniumhalogenid der Formel (III) eingesetzt. Zur weiteren Umsetzung entsprechend Stufe (b) wird das Reaktionsgemisch versetzt mit 1 Mol Säureakzeptor und 1 Mol Alkylmercaptan der Formel (V).

Das Reaktionsprodukt der Formel (I) kann nach üblichen Methoden aufgearbeitet werden. Vorzugsweise wird das Reaktionsprodukt, gegebenenfalls nach Abfiltrieren der anorganischen Salze, ohne weitere Aufarbeitung in die nächste Umsetzung eingesetzt.

Die nach dem erfindungsgemäßen Verfahren herstellbaren Verbindungen der Formel (I) sind wichtige Zwischenprodukte für die Synthese von Insektiziden. So lassen sich z.B. aus ihnen die substituierten N,N-Dimethyl-O-pyrimidinyl-carbaminsäureester, Verbindungen mit hoher insektizider Potenz herstellen (vgl. z.B. Deutsche Offenlegungsschrift Nr. 2 928 185).

Die Weiterverarbeitung der Verbindungen der Formel (I) zu bekannten Schädlingsbekämpfungsmitteln sei beispielhaft anhand des folgenden Schemas verdeutlicht:

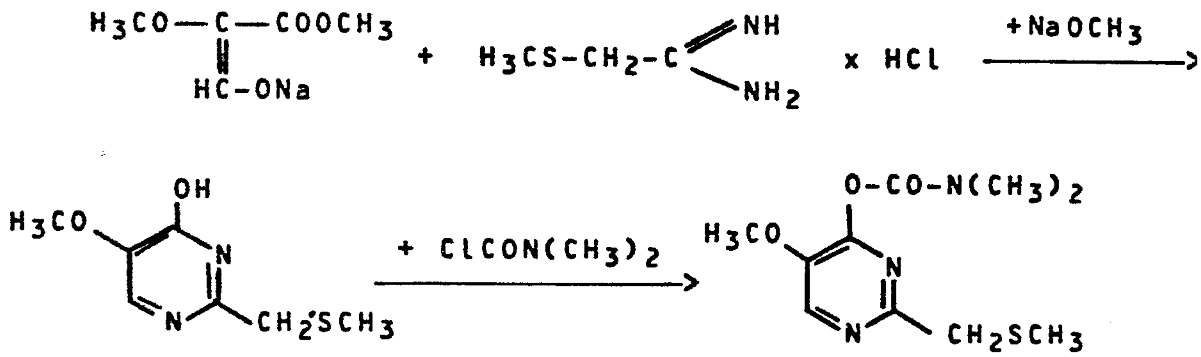

<u>Herstellungsbeispiel</u>

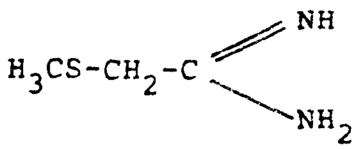 x HCl

Zu einer Mischung aus 100 kg Methanol (3125 Mol) und 4 kg (74 Mol) Natriummethylat werden bei 0°C bis 5°C 113 kg (1496 Mol) Chloracetonitril gegeben. Es wird 30 Minuten bei 0°C bis 5°C nachgerührt und anschließend mit 87,5 kg (1636 Mol) Ammoniumchlorid bei 0°C bis 5°C versetzt. Bei gleicher Temperatur wird erneut 30 Minuten nachgerührt, mit 316 kg 25 %ige methanolischer Natriummethylatlösung versetzt und anschließend werden 76 kg (1575 Mol) Methylmercaptan in den geschlossenen Reaktor gedrückt. Das Reaktionsgemisch wird 30 Minuten bei 0°C bis 5°C nachgerührt und vom Feststoff abfiltriert. Die gesamte Umsetzung wird unter Stickstoffatmosphäre durchgeführt. Aus dem Filtrat wird Methanol destillativ entfernt und das Reaktionsprodukt aus Aceton umkristallisiert.

Man erhält auf diese Weise 190 kg (90 % der Theorie) 2-Methylthio-acetamidin-Hydrochlorid vom Schmelzpunkt 70°C.

Patentansprüche

1. Verfahren zur Herstellung von Alkylthioacetamidinen der Formel (I)

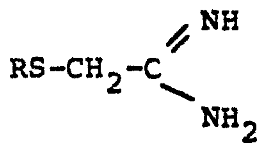 x $(HX)_n$ (I)

in welcher

R für Alkyl steht,

X für Halogen steht und

n für 0 oder 1 steht,

dadurch gekennzeichnet, daß man

(a) Chloracetonitril der Formel (II)

$ClCH_2CN$ (II)

mit Ammoniumhalogeniden der Formel (III)

$NH_4X$ (III)

in welcher

X die oben angegebene Bedeutung hat,

in Gegenwart von Katalysatoren und in Gegenwart von Verdünnungsmitteln bei Temperaturen zwischen -20°C und +30°C zu den Chloracetamidinen der Formel (IV)

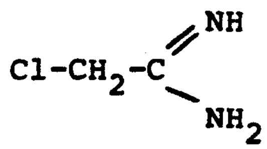 x $(HX)_n$ (IV)

in welcher

X und n die oben angegebenen Bedeutungen haben,

umsetzt und

(b) diese Verbindungen der Formel (IV) gegebenenfalls ohne Isolierung mit Alkylmercaptanen der Formel (V)

RSH (V)

in welcher

R die oben angegebene Bedeutung hat,

in Gegenwart von Säureakzeptoren und in Gegenwart von Verdünnungsmitteln, bei Temperaturen zwischen -20°C und +30°C und einem Druck zwischen 1 und 10 bar zu den Verbindungen der Formel (I) umsetzt.

2. Verfahren nach Anspruch 1, dadurch gekennzeichnet, daß man die Verbindungen der Formel (IV) nicht isoliert.

3. Verfahren nach Anspruch 1 oder 2, dadurch gekennzeichnet, daß man Verbindungen der Formel (I) herstellt, worin

R = $C_1$-$C_6$-Alkyl
X = Chlor oder Brom
und
n = 0 oder 1.

4. Verfahren nach Anspruch 1 bis 3, dadurch gekennzeichnet, daß man Verbindungen der Formel (I) herstellt, worin

R = Methyl, Ethyl, n-Propyl, i-Propyl, n-Butyl, i-Butyl, sec.-Butyl oder tert.-Butyl,

X = Chlor
und
n = 0 oder 1.

5. Verfahren nach Anspruch 1 bis 4, dadurch gekennzeichnet, daß man Methylthio-acetamidin-Hydrochlorid herstellt.

6. Verfahren nach Anspruch 1 bis 5, dadurch gekennzeichnet, daß man bei der Reaktionsstufe a) und bei der Reaktionsstufe b) einen Alkohol als Verdünnungsmittel einsetzt.

7. Verfahren nach Anspruch 1 bis 5, dadurch gekennzeichnet, daß als Katalysator in Reaktionsstufe a) ein Alkalialkoholat eingesetzt wird.

8. Verfahren nach Anspruch 1 bis 5, dadurch gekennzeichnet, daß als Säureakzeptor in Stufe b) ein Alkalialkoholat eingesetzt wird.

9. Verfahren nach Anspruch 1 bis 5, dadurch gekennzeichnet, daß die Umsetzung in beiden Stufen a) und b) bei Temperaturen zwischen -10°C und +20°C durchgeführt wird.

10. Verfahren zur Herstellung von Alkylthioacetamidinen der Formel (I)

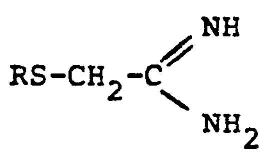 x $(HX)_n$ (I)

in welcher

R für Alkyl steht,

X für Halogen steht und

n für 0 oder 1 steht,

dadurch gekennzeichnet, daß man Chloracetamidine der Formel (IV)

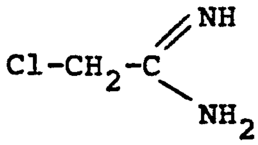 x $(HX)_n$ (IV)

in welcher

X und n die oben angegebenen Bedeutungen haben,

mit Alkylmercaptanen der Formel (V)

R-SH (V)

in welcher

R die oben angegebene Bedeutung hat,

in Gegenwart von Säureakzeptoren und in Gegenwart von Verdünnungsmitteln bei Temperaturen zwischen -20°C und +30°C und einem Druck zwischen 1 und 10 bar, umsetzt.